# EUROPEAN PATENT APPLICATION

(11) **EP 4 744 677 A1**
(43) Date of publication of application: **20.05.2026**
(21) Application number: 25174405.8
(22) Date of filing: 06.05.2025
(51) Int. Cl.: A61K 41/00, A61K 9/00, A61K 31/525, A61N 5/06

(54) **DRY EYE DISEASE TREATMENT THROUGH PHOTOSENSITIZER**

(30) Priority: 14.11.2024 US 202463720548 P
(71) Applicant: Universitatea de Medicina, Farmacie, Stiinte si Tehnologie "George Emil Palade" din Targu Mures, 540142 Targu Mures (RO); Queensland Eye Institute Foundation, Woolloongabba, 4102 (AU)
(72) Inventor: Azamfirei, Leonard, Livezeni (RO); Banescu, Claudia-Violeta, Corunca (RO); Chirila, Traian Vasile, Robertson, 4109 (AU); Manta, Alexandra Irina, Bulimba, 4171 (AU); Radford, Mark Hughes Burnard, Brisbane, 4000 (AU); Suzuki, Shuko, Ormeau, 4208 (AU)
(74) Representative: Enescu, Miruna

(57) **Abstract**

The present invention relates to a photosensitizer that initiates crosslinking in response to photoactivating radiation, for use in treating dry eye disease in a subject, wherein the photosensitizer is applied to at least a portion of the exposed palpebral conjunctiva of an eye of the subject, and the exposed palpebral conjunctiva is irradiated with photoactivating radiation to initiate crosslinking in the tarsal plate tissue at the base of the palpebral conjunctiva.

## Description

### METHOD OF TREATMENT FIELD OF THE INVENTION

The present invention relates generally to a method of treatment of dry eye disease. More particularly, this invention relates to a method of treatment of dry eye disease comprising exposure of a palpebral conjunctiva of an eye followed by transconjunctival irradiation in the presence of a photosensitizer to initiate crosslinking in tarsal plate tissue underlying the palpebral conjunctiva.

### BACKGROUND OF THE INVENTION

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavor to which this specification relates.

Located within the eyelids, the meibomian glands (MGs), known also as tarsal glands, are essentially sebaceous glands that are not accompanied by hairs. Their secretion (the "meibum") is an inordinately complex mixture of various lipids with other organic compounds, and represents the main component of the superficial layer of the tear film [Bron AJ, Tiffany JM, Gouveia SM, et al. "Functional aspects of the tear film lipid layer". Exp. Eye Res. 2004;78:347-360]. This layer fulfills two crucial needs, protection against evaporation of the aqueous phase and stabilization of tear film by lowering its surface tension. Each meibomian gland is constituted from a main duct (diameter, 100-150 µm; length, ~5.5 mm in the upper eyelid, ~2 mm in the lower eyelid), which is connected through ductules (diameter, 30-50 µm; length, ~150 µm) to circularly placed clumps of 10-15 secretory acini (clusters of meibocytes; diameter, 150-200 µm). Reported average numbers of glands are 31 for the upper eyelid and 26 for the lower eyelid. One end of each gland is blocked and the other opens at the eyelid margin as an orifice near the mucocutaneous junction where the delivery of meibum occurs [Jester JV, Nicolaides N, Smith RE. "Meibomian gland studies: histologic and ultrastructural investigations". Invest. Ophthalmol. Vis. Sci. 1981;20:537-547; Knop E, Knop N, Millar T, et al. "The International Workshop on meibomian gland dysfunction: Report of the Subcommittee on Anatomy, Physiology, and Pathophysiology of the Meibomian Gland". Invest. Ophthalmol. Vis. Sci. 2011;52:1938-1978].

In their native state, the meibomian glands are embedded within the tarsal plate (tarsus), which is a fibrocartilaginous entity able to provide both structural integrity to the eyelid and the stiffness needed for safeguarding its contour. The fibrous proteins in the tarsal plate's constitution include the collagen that imparts strength and the elastin that assures elasticity. The tarsal tissue consists mainly of collagens types I, III, and VI, and elastic networks of fibrillin and elastin fibers [Milz S, Neufang J, Higashiyama I, et al. "An immunohistochemical study of the extracellular matrix of the tarsal plate in the upper lid in human beings". J. Anat. 2005;206:37-45; Ezra DG, Beaconsfield M, Collin R. "Surgical anatomy of the upper eyelid: old controversies, new concepts". Expert Rev. Ophthalmol. 2009;4:47-57].

Meibomian gland dysfunction (MGD) is an umbrella term for a group of disorders, both congenital and acquired, which are associated with functional anomalies of the glands and can cause altered tear film, dry eye conditions and ocular surface disease [Knop E, Knop N, Millar T, et al. "The International Workshop on meibomian gland dysfunction: Report of the Subcommittee on Anatomy, Physiology, and Pathophysiology of the Meibomian Gland". Invest. Ophthalmol. Vis. Sci. 2011;52:1938-1978; Amano S, Shimazaki J, Yokoi N, et al. "Meibomian gland dysfunction clinical practice guidelines". Jpn. J. Ophthalmol. 2023;67:448-539]. According to the Tear Film & Ocular Surface Society (TFOS), dry eye disease (DED) is defined as "a multifactorial disease of the ocular surface characterized by a loss of homeostasis of the tear film, and accompanied by ocular symptoms, in which tear film instability and hyperosmolarity, ocular surface inflammation and damage, and neurosensory abnormalities play etiological roles" [Craig JP, Nichols KK, Akpek EK, et al. "TFOS DEWS II Definition and classification report". Ocul. Surf. 2017;15:276-283]. The prevalence of DED around the world varies from 5% to 50% and its economic burden and impact on vision, quality of life and work productivity are substantial [Stapleton F, Alves M, Bunya VY, et al. "TFOS DEWS II Epidemiology report". Ocul. Surf. 2017;15:334-365]. DED and impaired vision result from disturbances in the natural tear film that is bathing the ocular surface. The primary culprit behind this disruption is a heightened evaporation of the aqueous phase of the tears due to alterations in meibum secretion that are caused by MGD. Indeed, MGD is currently recognized as the leading cause of evaporative DED [Messmer E. "The pathophysiology, diagnosis, and treatment of dry eye disease". Dtsch. Arztebl. Int. 2015;112:71-82; Baudouin C, Messmer EM, Aragona P, et al. "Revisiting the vicious circle of dry eye disease: a focus on the pathophysiology of meibomian gland dysfunction". Br. J. Ophthalmol. 2016;100:300-306; Chhadva P, Goldhardt R, Galor A. "Meibomian gland disease. The role of gland dysfunction in dry eye disease". Ophthalmology 2017;124(11 Suppl):S20-S26; Ekin MA, Ugurlu SK, Imre SS, et al. "The role of meibomian gland dysfunction on the development of dry eye disease in patients with facial nerve palsy". Arq. Bras. Oftalmol. 2022;85:128-135; Sheppard JD, Nichols KK. "Dry eye disease associated with Meibomian gland dysfunction: focus on tear film characteristics and the therapeutic landscape". Ophthalmol. Ther. 2023;12:1397-1418]. The vast majority of dry eye patients have evaporative DED.

Smooth and uniform delivery of the meibum through the ductal orifices positioned onto the eyelid margins is an important factor able to improve the function of MGs by reducing detrimental effects on the tear film that may contribute to the development of DED. The process of meibum delivery is governed by the mechanical characteristics of the tissues surrounding the encapsulated MGs. More precisely, the concerted mechanical action of two eyelid muscles, external to the tarsus, is essential for the delivery process, these muscles being the pretarsal part of the palpebral portion of orbicularis oculi muscle, and its most superficial fibers at the lid margin known as the muscle of Riolan [Linton RG, Curnow DH, Riley WJ. "The meibomian glands. An investigation into the secretion and some aspects of the physiology". Br. J. Ophthalmol. 1961;45:718-723; Dailey RA, Wobig JL. "Eyelid anatomy". J. Dermatol. Surg. Oncol. 1992;18:1023-1027; Knop E, Knop N, Millar T, et al. "The International Workshop on meibomian gland dysfunction: Report of the Subcommittee on Anatomy, Physiology, and Pathophysiology of the Meibomian Gland". Invest. Ophthalmol. Vis. Sci. 2011;52:1938-1978; Gao Q, Xu P, Hu S, et al. "The micro-structure and biomechanics of eyelid tarsus". J. Biomech. 2022;123:110911]. It is accepted that the orbicularis muscle compresses the plate with the enclosed glands promoting the secretion of meibum through a squeeze-driving ("milking") action. In its turn, the contraction of the muscle of Riolan can compress the terminal part of the glands and contribute to the delivery of meibum, and in addition prevents its outflow between blinks. MGs are embedded in the tarsal fibrocartilage, where they are in close apposition with the fibrous elastin-collagen system. This network system plays a passive, but important, role of transmitting the muscle-generated compressive forces to individual glands through an effect of homogeneous distribution of the forces. In other words, the tarsal fibrous network acts as a semisolid matrix within which the forces generated by the neighboring muscles can be exerted on the glands without being in direct contact with them.

As shown above, the mechanobiology of MGs reveals the importance of a normal tarsal plate, where the elastin and collagen fibers can display unaltered their mechanical properties (strength, stiffness) to assist in the meibum delivery process. Any disturbances in the structure and properties of the periglandular elastin-collagen continuum is reflected eventually in the deficient delivery of meibum and its consequences. However, advanced laxity and loss of tone is a characteristic feature of aging ocular adnexal tissues, including the eyelids. Tarsal laxity as such is also a salient etiologic aspect in a number of identifiable abnormalities of the eyelid including lax eyelid syndromes (with floppy eyelid syndrome as the prominent example), ptosis (involutional and mechanical), involutional and paralytic ectropion, involutional entropion, distension due to eyelid tumors, blepharochalasis, facial dysmorphism syndromes, and hypermobility syndromes. In all such conditions, decline in elastin and collagen production, alongside with alterations in their organization and quality have been demonstrated [Ezra DG, Ellis JS, Gaughan C, et al. "Changes in tarsal plate fibrillary collagens and elastic fibre phenotype in floppy eyelid syndrome". Clin. Exp. Ophthalmol. 2011;39:564-571; Netland PA, Sugrue SP, Albert DM, et al. "Histopathologic features of the floppy eyelid syndrome. Involvement of tarsal elastin". Ophthalmology 1994;101:174-181; Damasceno RW, Osaki MH, Dantas PE, et al. "Involutional ectropion and entropion: clinicopathologic correlation between horizontal eyelid laxity and eyelid extracellular matrix". Ophthal. Plast. Reconstr. Surg. 2011;27:321-326; Sutula FC. "Histological changes in congenital and acquired blepharoptosis". Eye 1988;2:179-184].

An interrelation between eyelid laxity (either floppy eyelid syndrome or other conditions) and MGD/DED has been conclusively demonstrated [Gonnering RS, Sonneland PR. "Meibomian gland dysfunction in floppy eyelid syndrome". Ophthalm. Plast. Reconstr. Surg. 1987;3:99-103; Liu DT-S, Di Pascuale M, Sawai J, et al. "Tear film dynamics in floppy eyelid syndrome". Invest. Ophthalmol. Vis. Sci. 2005;46:1188-1194; Mastrota KM. "Impact of floppy eyelid syndrome in ocular surface and dry eye disease". Optom. Vis. Sci. 2008;85:814-816; Ansari Z, Singh R, Alabiad C, et al. "Prevalence, risk factors, and morbidity of eye lid laxity in a veteran population". Cornea 2015;34:32-36; Chhadva P, McClellan AL, Alabiad CR, et al. "Impact of eyelid laxity on symptoms and signs of dry eye disease". Cornea 2016;35:531-535]. Weakening of fibrous elastin-collagen network in the tarsal plate compromises its roles of supporting MGs and in contributing favorably to the meibum delivery process, which is actually effected by muscles and mediated by the tarsal elastin and collagen. Lack of mechanical support can lead to both acinar deformity and contortion of the ductules, which further compromise the transport and delivery of meibum. The process becomes akin to pushing a liquid through a kinked hose. Consequences include altered tear dynamics, disruption of the lid ocular surface interface, and irregular wetting. Ultimately, a twisted ductal system may result in blockage of meibum circulation contributing to pressure atrophy of acini and to eventual collapse of glands structure.

The interplay between the tarsal elastin-collagen network and the MG function is critical for managing DED. There is a need for non-invasive methods that treat DED.

### SUMMARY OF THE INVENTION

This invention is predicated in part on the discovery that exposure of a palpebral conjunctiva (also known as the tarsal conjunctiva), which is the mucous membrane lining the inside of an eyelid, to radiation in the presence of a photosensitizer initiates crosslinking within the underlying tarsal plate, and particularly crosslinking of collagen in the tarsal plate tissue, and that such crosslinking restores and/or increases meibomian gland function. Without wishing to be bound by theory, the inventors have proposed that the crosslinking of collagen in the tarsal plate leads to a stronger embedding of meibomian glands into the tarsal elastin-collagen network and the straightening of their main duct and ductules. The inventors have proposed that this will facilitate improved meibum delivery to the eye surface from the meibomian glands. Accordingly, the inventors have conceived that the application of a photosensitizer that initiates crosslinking in response to photo-activating radiation to exposed palpebral conjunctiva, followed by irradiation with photo-activating radiation may be used to treat dry eye disease. This method provides a non-invasive method for treatment of dry eye disease.

Accordingly, in one aspect, there is provided a method of treating dry eye disease in a subject comprising:
a) exposing a palpebral conjunctiva of an eye;
b) applying to at least part of the exposed palpebral conjunctiva a photosensitizer that initiates crosslinking in response to photo-activating radiation; and
c) irradiating the exposed palpebral conjunctiva with photo-activating radiation to initiate crosslinking in tarsal plate tissue underlying the palpebral conjunctiva.

In some embodiments, the photosensitizer is in solubilized form. In some embodiments, the photosensitizer is selected from the group consisting of riboflavin, acridine orange, Quantacure QTX, protoporphyrin IX and pharmaceutically acceptable salts, derivatives and solvates thereof. In some embodiments, the photosensitizer absorbs radiation at a wavelength in the range of from about 300 to about 500 nm. In particular embodiments, the photosensitizer is riboflavin or a pharmaceutically acceptable salt, derivative or solvate thereof; including riboflavin 5'-phosphate or a pharmaceutically acceptable salt or solvate thereof.

In some embodiments, the photo-activating radiation is radiation with a wavelength in the range of from about 300 to about 500 nm. In some embodiments, the photo-activating radiation is UV-A radiation, such as radiation with a wavelength of about 365 nm.

In some embodiments, the palpebral conjunctiva is exposed by everting an upper or lower eyelid of the subject to expose the palpebral conjunctiva, such as by using a lid retractor.

In particular embodiments, the method further includes delivery of O₂ gas at a site of irradiation.

Also provided, in another aspect, is a use of a photosensitizer that initiates crosslinking in response to photo-activating radiation in the manufacture of a medicament for treating dry eye disease in a subject, wherein the photosensitizer is to be applied to at least part of an exposed palpebral conjunctiva of an eye of the subject, and the exposed palpebral conjunctiva is to be irradiated with photo-activating radiation to initiate crosslinking in tarsal plate tissue underlying the palpebral conjunctiva.

In a further aspect, there is provided a use of a photosensitizer that initiates crosslinking in response to photo-activating radiation for treating dry eye disease in a subject, wherein the photosensitizer is to be applied to at least part of an exposed palpebral conjunctiva of an eye of the subject, and the exposed palpebral conjunctiva is to be irradiated with photo-activating radiation to initiate crosslinking in tarsal plate tissue underlying the palpebral conjunctiva.

In yet another aspect, there is provided a photosensitizer that initiates crosslinking in response to photo-activating radiation for use in treating dry eye disease in a subject, wherein the photosensitizer is applied to at least part of an exposed palpebral conjunctiva of an eye of the subject, and the exposed palpebral conjunctiva is irradiated with photo-activating radiation to initiate crosslinking in tarsal plate tissue underlying the palpebral conjunctiva.

### BRIEF DESCRIPTION OF THE DRAWINGS

Figure 1 is a diagram outlining the method of the invention. Radiation and the photosensitizer penetrate across the palpebral conjunctiva (p.c.) to reach the tarsus surrounding the meibomian glands (MG).
Figure 2 comprises histological micrographs (magnification x20) of the tarsal tissue in ovine eyelids showing (A) normal non-irradiated tarsal conjunctiva; (B) conjunctiva exposed to an irradiance of 45 mW/cm²; (C) damaged conjunctiva that was exposed to an irradiance of 250 mW/cm².
Figure 3 shows bar graphs of the tensile properties of ovine tarsoconjunctival tissue before and after UV-A irradiation. A: Young's modulus; B: stress at 10% elongation (n=6).
Figure 4 is the real-time PCR gene expression workflow.
Figure 5 is a multicomponent plot of the amplification by realtime PCR of ATM gene and the control reference gene in the UV-exposed and control group samples. 1: amplification of control reference; 2: amplification of investigated gene (ATM); 3: negative controls - no amplification.

### DETAILED DESCRIPTION OF THE INVENTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (i.e. to at least one) of the grammatical object of the article. By way of example, "an element" means one element or more than one element.

By "about" is meant a quantity, level, value, number, frequency, percentage, dimension, size, amount, weight or length that varies by as much 15, 14, 13, 12, 11, 10, 9, 8, 7, 6, 5, 4, 3, 2 or 1 % to a reference quantity, level, value, number, frequency, percentage, dimension, size, amount, weight or length.

The term "agent" includes a compound that induces a desired pharmacological and/or physiological effect. The term also encompasses pharmaceutically acceptable and pharmacologically active ingredients of those compounds specifically mentioned herein including but not limited to salts, esters, amides, prodrugs, active metabolites, analogues and the like. When the above term is used, then it is to be understood that this includes the active agent *per se* as well as pharmaceutically acceptable, pharmacologically active salts, esters, amides, prodrugs, metabolites, analogues, etc.

As used herein, the term "and/or" refers to and encompasses any and all possible combinations of one or more of the associated listed items, as well as the lack of combinations when interpreted in the alternative (or).

The phrase "aqueous carrier" is used herein to refer to a liquid aqueous diluent, wherein the aqueous carrier includes, but is not limited to, water, saline, aqueous buffer and aqueous solutions comprising water soluble or water miscible additives such as glucose or glycerol. The aqueous carrier may also be in the form of an oil-in-water emulsion.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. Thus, the use of the term "comprising" and the like indicates that the listed integers are required or mandatory, but that other integers are optional and may or may not be present. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified for the listed elements. Thus, the phrase "consisting essentially of" indicates that the listed elements are required or mandatory, but that other elements are optional and may or may not be present depending upon whether or not they affect the activity or action of the listed elements.

As used herein, the terms "condition" or "disease" refers to an abnormality in the physical state of the body as a whole or one of its parts.

When used herein, the term "crosslinker" or "crosslinking agent" refers to a chemical moiety that can chemically join two or more molecules, for example by covalent bonding or ionic bonding, preferably by covalent bonding. An example of a crosslinking agent is O₂ which acts as a crosslinking agent when in the form of its high energy singlet state. A crosslinker or crosslinking agent will preferably be pharmaceutically acceptable.

By "derivative" is meant a molecule, such as a small molecule, that has been derived from the basic molecule by modification, for example by conjugation or complexing with other chemical moieties as would be understood in the art. The term "derivative" also includes within its scope alterations that have been made to a parent molecule including additions or deletions that provide for functionally equivalent molecules.

The term "dry eye disease" as used herein refers to a disease of the ocular surface characterized by a loss of homeostasis of the tear film, and accompanied by ocular symptoms, in which tear film instability and hyperosmolarity, ocular surface inflammation and damage, and neurosensory abnormalities play etiological roles. In some embodiments, the dry eye disease is evaporative dry eye disease, which is due to a deficient team film lipid layer, which increases tear evaporation. Evaporative dry eye disease is typically caused by or associated with meibomian gland dysfunction. In some embodiments, the dry eye disease is associated with meibomian gland dysfunction. Symptoms of dry eye disease include, for example, ocular discomfort; feeling or grittiness or pain; visual disturbance, including intermittent blurry vision which resolves with blinking, rubbing of the eyes or instillation of lubricating eye drops; light sensitivity; tearing; red eyes; tear film instability; inflammation of the ocular surface; increased tear film osmolarity; and the like. Signs on ophthalmic examination may include, for example, conjunctival injection, lid margin hyperaemia, decreased tear meniscus, presence punctate epithelial erosions or mucus strands on the cornea, meibomian gland dysfunction and corneal vascularization. The presence of dry eye disease may be confirmed using the Schirmer Test <10 mm at 5 minutes, ocular surface staining with fluorescein, pr determining that the subject has a tear break-up time of less than 10 sec or a tear osmolarity <308 mOsm/L.

By "effective amount", in the context of treating dry eye disease is meant the application of an amount of an agent or composition to an individual in need of such treatment, that is effective for preventing incurring a symptom, holding in check such symptom(s) and/or treating existing symptom(s) of dry eye disease. The effective amount will vary depending upon the health and physical condition of the individual to be treated, the taxonomic group of individual to be treated, the formulation of the composition, the assessment of the medical situation, and other relevant factors. It is expected that the amount will fall in a relatively broad range that can be determined through routine trials.

By "pharmaceutically acceptable carrier" is meant a pharmaceutical vehicle comprised of a material that is not biologically or otherwise undesirable, i.e., the material may be administered to a subject along with the selected active agent without causing any or a substantial adverse reaction.

Similarly, a "pharmacologically acceptable" salt, ester, amide, prodrug, compound or derivative of a compound as provided herein is a salt, ester, amide, prodrug, compound or derivative that this not biologically or otherwise undesirable.

The term "photo-activating radiation" when used herein refers to radiation that can activate a photosensitizer to produce a chemical change in another molecule. Suitably the photosensitizer absorbs radiation at a wavelength of the photo-activating radiation. In some embodiments the radiation is radiation at a wavelength in the range of from about 300 to about 500 nm, especially UV-A radiation.

When used herein, the term "photosensitizer" refers to a molecule that, on irradiation by photo-activating radiation, produces a chemical change in another molecule through a photochemical process. Examples of "another molecule" include, for example, a crosslinker or crosslinking agent such as O₂. A photosensitizer may convert O₂ molecules from the normal O₂ triplet state to a more energetic singlet state that can initiate crosslinking, for example in tissue molecules or macromolecules. Further examples of "another molecule" include tissue molecules or macromolecules, including collagen macromolecules. A photosensitizer, after exposure to radiation and transition to a more energetic state, may also produce a chemical change in collagen and/or other tissue molecules and initiate or generate crosslinking in the tissue. The skilled person will appreciate that optimum results will be achieved when the selected photosensitizer absorbs radiation at a wavelength of the photo-activating radiation. The absorption wavelength(s) of a photosensitizer can be determined by ultraviolet/visible (UV/VIS) spectrophotometry using a commercially available UV/VIS spectrophotometer in accordance with well known procedures. A photosensitizer will preferably be pharmaceutically acceptable, non-irritant and non-toxic.

As used herein, the term "salts", "derivative" and "solvate" include any pharmaceutically acceptable salt, derivative, or solvate or any other compound which, upon administration to the recipient, is capable of providing (directly or indirectly) a desired photosensitizer. Suitable pharmaceutically acceptable derivatives include esters, such as phosphate esters. Suitable pharmaceutically acceptable salts include salts of pharmaceutically acceptable inorganic acids such as hydrochloric, sulfuric, phosphoric, nitric, carbonic, boric, sulfamic and hydrobromic acids, or salts of pharmaceutically acceptable organic acids such as acetic, propionic, butyric, tartaric, maleic, hydroxymaleic, fumaric, citric, lactic, mucic, gluconic, benzoic, succinic, oxalic, phenylacetic, methanesulfonic, toluenesulfonic, benzenesulfonic, salicylic, sulfanilic, aspartic, glutamic, edetic, stearic, palmitic, oleic, lauric, pantothenic, tannic, ascorbic and valeric acids. Base salts include, but are not limited to, those formed with pharmaceutically acceptable cations, such as sodium, potassium, lithium, calcium, magnesium, ammonium and alkylammonium, particularly sodium. Also, basic nitrogen-containing groups may be quaternized with such agents as lower alkyl halides, such as methyl, ethyl, propyl and butyl chlorides, bromides and iodides; dialkyl sulfates such as dimethyl and diethyl sulfate; and others. Pharmacologically acceptable solvates are known in the art, and include hydrates and alcoholates. Suitably, pharmaceutically acceptable solvates include hydrates, for example monohydrates, dihydrates and trihydrates. The skilled person will understand that a photosensitizer may be in the form of a pharmaceutically acceptable salt, and/or a solvate and/or a derivative, for example riboflavin 5'-phosphate monosodium salt dihydrate and the like. The preparation of salts, derivatives and solvates can be carried out by methods well known in the art. Lists of suitable salts are found in, for example, Allen LV (Ed.) "Remington: The Science and Practice of Pharmacy". The Pharmaceutical Press, London, 2012, 22nd edition; Stahl PH and Wermuth CG (Eds.) "Pharmaceutical Salts: Properties, Selection, and Use". Wiley-VCH, Germany, 2002, 2nd edition; and Berge SM et al. "Pharmaceutical salts". J. Pharm. Sci. 1977;66:1-19, each of which is incorporated herein by reference in its entirety.

The term "simultaneously" denotes that the two agents are applied at substantially the same time.

As used herein, the phrase "solubilized form" refers to a form where a compound, such as a photosensitizer, is dissolved in a liquid such that a solution comprising a uniform distribution of the compound is obtained which is substantially free of solid compound. In some embodiments, the liquid is an aqueous carrier as described herein.

The term "subject" or "individual" as used herein refers to a vertebrate subject, particularly a mammalian or avian subject, for whom therapy or prophylaxis is desired. Suitable subjects include, but are not limited to, primates; avians (birds); livestock animals such as sheep, cows, horses, deer, donkeys and pigs; laboratory test animals such as rabbits, mice, rats, guinea pigs and hamsters; companion animals such as cats and dogs; and captive wild animals such as foxes, deer and dingoes. In particular embodiments, the subject is a primate, suitably a human. However, it will be understood that the aforementioned terms do not imply that symptoms are present.

As used herein, the terms "treatment", "treating", and the like, refer to obtaining a desired pharmacologic and/or physiologic effect. The effect may be therapeutic in terms of a partial or complete cure for a disease, disorder or condition and/or adverse effect attributable to the disease, disorder or condition. These terms also cover any treatment of a condition or disease in a subject, particularly in a human, and include: (a) inhibiting the disease, disorder or condition, i.e. arresting its development; (b) relieving the disease, disorder or condition, i.e. causing regression of the disease, disorder or condition; or (c) relieving one or more symptoms of the disease, disorder or condition.

As used herein "water soluble form" refers to a chemical and/or physical form of a compound, such as a photosensitizer, where the compound or a salt, derivative, solvate and/or polymorph thereof has sufficient solubility in water at ambient temperature to achieve a concentration of from about 0.01 to about 20% w/v. Solubility can be determined using methods well known in the art.

Each embodiment described herein is to be applied *mutatis mutandis* to each and every embodiment unless specifically stated otherwise.

### 2. Methods of Treatment

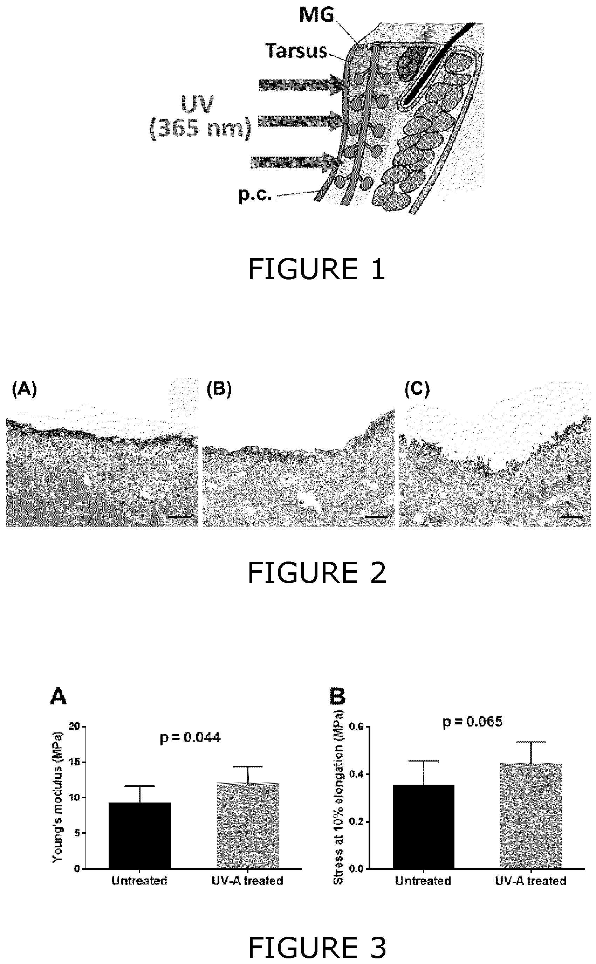

The present invention is based, in part, on the identification that exposure of a palpebral conjunctiva to radiation in the presence of a photosensitizer initiates crosslinking within the underlying tarsal plate, and particularly crosslinking of collagen in the tarsal plate tissue, and that such crosslinking restores and/or increases meibomian gland function. Thus, the inventors conceived that treatment of dry eye disease or inhibition of the progression of dry eye disease may be achieved by irradiating the palpebral conjunctiva with photo-activating radiation in the presence of a photosensitizer. A diagram demonstrating the application of the radiation to the palpebral conjunctiva is provided in Figure 1.

Accordingly, the present invention provides a method of treating dry eye disease in a subject comprising, consisting or consisting essentially of:
a) exposing a palpebral conjunctiva of an eye;
b) applying to at least part of the exposed palpebral conjunctiva a photosensitizer that initiates crosslinking in response to photo-activating radiation; and
c) irradiating the exposed palpebral conjunctiva with photo-activating radiation to initiate crosslinking in tarsal plate tissue underlying the palpebral conjunctiva.

The methods may also be useful for inhibiting the progression of dry eye disease.

In some embodiments, the dry eye disease is evaporative dry eye disease. In some embodiments, the dry eye disease is associated with meibomian gland dysfunction.

The methods are useful for treating mild, moderate or severe dry eye disease. In some embodiments, the dry eye disease is moderate or severe dry eye disease.

The methods of the invention may be applied to the palpebral conjunctiva of an upper eyelid, a lower eyelid or both eyelids, subsequently or simultaneously. The methods may be applied to one or both eyes of a subject, individually, subsequently or simultaneously. In particular embodiments, the methods are applied to both eyes of a subject.

A person of skill in the art will be well aware of suitable methods for exposing a palpebral conjunctiva of an eye of the subject. For example, the palpebral conjunctiva may be exposed by everting an eyelid of the subject, such as an upper and/or lower eyelid of the subject. Suitable techniques include, but are not limited to, a device such as a lid retractor, or an item pressed on the eyelid at the upper edge of the tarsal plate for the superior eyelid or the lower edge for the inferior eyelid, such as a cotton bud, a finger or a glass rod with manual eversion of the eyelid to expose the palpebral conjunctiva. Suitable techniques are discussed, for example, in Wolffsohn JS, Tahhan M, Vidal-Rohr, et al. "Best technique for upper lid eversion". Contact Lens and Anterior Eye. 2019;42:666-669. In some embodiments, the palpebral conjunctiva is exposed using a lid retractor.

An exemplary procedure for everting an eyelid of a subject includes:
a) instructing the subject to look down without closing the eyes;
b) pulling the eyelid slightly downward and away from the globe of the eye to create tension;
c) if using a finger or cotton-tipped applicator, placing the finger or cotton-tipped applicator at the upper edge of the tarsal plate through the eyelid, approximately 10 mm above the eyelid margin on the upper eyelid or 4 mm below the eyelid margin on the lower eyelid; and
d) pressing gently on the tarsal plate edge through the eyelid while lifting the eyelid margin upward and over the pressure point to expose the palpebral conjunctiva overlying the entire tarsal plate.

The photosensitizer is suitably pharmaceutically acceptable, substantially non-toxic and substantially non-irritant. In particular embodiments, the photosensitizer is water soluble. The skilled person will readily understand that different photosensitizers will absorb photosensitizing radiation of specific wavelengths according to the chemical structure of the chromophore, and will be able to match the photosensitizer to the appropriate wavelength of photosensitizing radiation. In some embodiments, the photosensitizer absorbs radiation in the ultraviolet region of the electromagnetic spectrum, especially radiation at a wavelength in the range of from about 300 to about 500 nm (and all integers therebetween) or a wavelength in the range of from about 300 to about 400 nm. In preferred embodiments, the photosensitizer absorbs long wavelength ultraviolet radiation e.g. UV-A radiation (about 320 to about 400 nm). In particular embodiments, the photosensitizer absorbs radiation at a wavelength of about 320, 325, 330, 335, 340, 345, 350, 355, 360, 365, 370, 375, 380, 385, 390, 395 or 400 nm; especially about 365 nm. In some embodiments, the photosensitizer absorbs radiation at a wavelength of about 450 to about 495 nm (blue light). In other embodiments, the photosensitizer absorbs radiation at a wavelength of about 495 to about 570 nm (green light). In preferred embodiments, the photosensitizer has regulatory approval for food and/or drug use. While the use of more than one photosensitizer is encompassed herein, in particular embodiments, there is only a single photosensitizer molecule present.

In some embodiments, the photosensitizer comprises riboflavin or a pharmaceutically acceptable salt, derivative or solvate thereof. Riboflavin is also known as vitamin B₂, and has the IUPAC name 7,8-dimethyl-10-[(25,35,4R)-2,3,4,5-tetrahydroxypentyl]benzo[g]pteridine-2,4-dione. While any form of riboflavin is suitable, in preferred embodiments, riboflavin is in a water soluble form, for example as a water soluble derivative, salt or solvate, such as an alkali metal salt of riboflavin 5'-phosphate. In particular embodiments, the photosensitizer comprises a sodium salt of riboflavin 5'-phosphate or a pharmaceutically acceptable solvate thereof, such as riboflavin 5'-phosphate monosodium salt. When in the form of a solvate, the solvate is preferably a hydrate.

In some embodiments, the photosensitizer is rose bengal (4,5,6,7-tetrachloro-2',4',5',7'-tetraiodofluorescein disodium salt). Preferably rose bengal is used in conjunction with irradiation by green light, i.e. a radiation wavelength of about 495 to about 570 nm.

In some embodiments, the photosensitizer is selected from the group consisting of lucigenin, acridine orange, riboflavin, Quantacure QTX (2-hydroxy-3-(3,4-dimethyl-9-oxo-9H-thioxanthen-2-yloxy)-N,N,N-trimethyl-1-propanaminium chloride), erythrosine B, protoporphyrin IX and pharmaceutically acceptable salts, derivatives and solvates thereof; especially riboflavin, acridine orange, Quantacure QTX, protoporphyrin IX or pharmaceutically acceptable salts, derivatives and solvates thereof. In such embodiments, the irradiation may have a wavelength of greater than about 300 nm, such as from about 300 nm to about 500 nm (e.g. UV-A or blue light).

In some embodiments, the photosensitizer is selected from the group consisting of Lissamine green B, Brilliant blue G, trypan blue, rose bengal and pharmaceutically acceptable salts, derivatives and solvates thereof. In such embodiments, the irradiation may have a wavelength of greater than about 400 nm, such as from about 495 nm to about 570 nm (e.g. green light).

In some embodiments, the photosensitizer is selected from the group consisting of riboflavin, rose bengal, lucigenin, acridine orange, Quantacure QTX, Lissamine green B, Brilliant blue G, trypan blue, erythrosine B, protoporphyrin IX and pharmaceutically acceptable salts, derivatives and solvates thereof; especially riboflavin, acridine orange, Quantacure QTX, protoporphyrin IX, lucigenin, rose bengal, erythrosine B and pharmaceutically acceptable salts, derivatives and solvates thereof; more especially riboflavin, acridine orange, Quantacure QTX, protoporphyrin IX and pharmaceutically acceptable salts, derivatives and solvates thereof. In preferred embodiments, the photosensitizer is riboflavin or a pharmaceutically acceptable salt, derivative or solvate thereof, such as riboflavin 5'-phosphate or a pharmaceutically acceptable salt or solvate thereof.

In some embodiments, the photosensitizer is applied to at least part of the exposed palpebral conjunctiva. Suitably the photosensitizer is applied to at least 25%, at least 50%, at least 75%, at least 80% or at least 90% of the surface of the exposed palpebral conjunctiva. Preferably the photosensitizer is applied to substantially the entire surface of the exposed palpebral conjunctiva.

In some embodiments, the photosensitizer is in solubilized form. Preferably, the photosensitizer is water soluble.

While the photosensitizer may be applied in a number of suitable forms, in particular embodiments, the photosensitizer is formulated in a carrier, such as a liquid or gel carrier. In such embodiments, the photosensitizer may be administered in a pharmaceutical composition comprising the photosensitizer and a pharmaceutically acceptable carrier.

The photosensitizer is preferably in a form suitable for topical application to the palpebral conjunctiva, such as an emulsion, solution or gel; especially a solution. Techniques for formulation and administration may be found in, for example, Allen LV (Ed.) "Remington: The Science and Practice of Pharmacy". The Pharmaceutical Press, London, 2012, 22nd edition.

In some embodiments, the photosensitizer is formulated as an aqueous composition, for example an aqueous solution, an aqueous gel, or an oil in water emulsion, preferably as an aqueous solution. In particular embodiments, the photosensitizer is present in the composition in an amount of about 0.01 to about 20% w/v (and all integers therebetween), such as about 0.1% to 10% w/v, about 0.1% to 5% w/v, about 0.1% to 2% w/v, about 0.1% to 1% w/v, about 0.1% to 0.5% w/v or about 0.1% to 0.3% w/v. In some embodiments, the photosensitizer is present in an amount of about 0.1%, 0.2% or 0.3 w/v.

In some embodiments, the carrier is an aqueous carrier. The aqueous carrier is preferably a pharmaceutically acceptable aqueous carrier. A variety of pharmaceutically acceptable aqueous carriers well known in the art may be used. Exemplary aqueous carriers include, but are not limited to, saline, water, aqueous buffer, an aqueous solution comprising water and a miscible solvent, and combinations thereof. In particular embodiments, the aqueous carrier is saline. When saline is used, it is preferably isotonic for the point of administration. For example, in some embodiments the saline comprises 0.15 to 8% w/v sodium chloride (and all one hundredth integer percentages therebetween); especially 0.18% to 7% w/v, 0.22% to 5% w/v or 0.45% to 3% w/v sodium chloride; more especially 0.5 to 2% w/v or 0.65% to 1.5% w/v sodium chloride; most especially about 0.9% w/v sodium chloride.

In some embodiments where the aqueous carrier is not isotonic, for example water, the composition may contain a tonicity agent. Any pharmaceutically acceptable tonicity agent well known in the art may be used. Suitable tonicity agents include, but are not limited to, boric acid, sodium acid phosphate buffer, sodium chloride, glucose, trehalose, potassium chloride, calcium chloride, magnesium chloride, poly(propylene glycol), glycerol, mannitol, or salts or combinations thereof. The tonicity agent may be present in the composition in an amount that provides isotonicity with the point of administration, for example in the range of from 0.02 to 15% w/v (and all one hundredth integer percentages therebetween).

In some embodiments the carrier is a buffer, wherein the buffer maintains a pH in the range of from 6 to 8, 6.5 to 7.5 or about 7. Suitable buffering agents include, but are not limited to, acetic acid, citric acid, sodium metabisulfite, histidine, sodium bicarbonate, sodium hydroxide, boric acid, borax, alkali metal phosphates, phosphate or citrate buffers, or combinations thereof. The buffering agent may be present in the composition in an amount suitable to maintain the desired pH.

The composition may further comprise a rheology modifier. The rheology modifier may be used to alter the surface tension and flow of the composition. Suitable rheology modifiers are well known in the art. For example, the rheology modifier may be selected from, but is not limited to, hyaluronic acid, chitosan, poly(vinyl alcohol), poly(ethylene glycol), poly(vinyl pyrrolidone), dextran, methylcellulose, hydroxypropylmethylcellulose, hydroxyethylcellulose, hydroxypropylcellulose, hydroxypropyl guar, acrylates such as Carbopol polymers, poloxamers, gum arabic, xanthan gum, guar gum, locust bean gum, carboxymethylcellulose, alginate, starch (from rice, corn, potato or wheat), carrageenan, konjac, aloe vera gel, agarose, pectin, tragacanth, curdlan gum, gellan gum, scleroglucan, and derivatives and combinations thereof. The rheology modifier should be present in an amount sufficient to obtain the desired viscosity of the composition. The rheology modifier may be present in an amount in the range of from about 0.5% to 5% w/v of the composition (and all one tenth integer percentages therebetween).

The composition may further comprise a surfactant. A variety of pharmaceutically acceptable surfactants well known in the art may be used. Exemplary surfactants include, but are not limited to, surfactants of the following classes: alcohols; amine oxides; block polymers; carboxylated alcohol or alkylphenol ethoxylates; carboxylic acids/fatty acids; ethoxylated arylphenols; ethoxylated fatty esters, oils, fatty amines or fatty alcohols such as cetyl alcohol; fatty esters; fatty acid methyl ester ethoxylates; glycerol esters such as glycerol monostearate; glycol esters; lanolin-based derivatives; lecithin or derivatives thereof; lignin or derivatives thereof; methyl esters; monoglycerides or derivatives thereof; poly(ethylene glycol)s; poly(propylene glycol)s; alkylphenol poly(ethylene glycol)s; alkyl mercaptan poly(ethylene glycol)s; poly(propylene glycol) ethoxylates; poly(ethylene glycol) ethers such as Cetomacrogol 1000; polymeric surfactants; propoxylated and/or ethoxylated fatty acids, alcohols or alkylphenols; protein-based surfactants; sarcosine derivatives; sorbitan derivatives such as polysorbates; sorbitol esters; esters of sorbitol polyglycol ethers; fatty acid alkylolamides; N-alkylpolyhydroxy fatty acid amide; N-alkoxypolyhydroxy fatty acid amide; alkyl polyglycosides; quaternary ammonium compounds such as benzalkonium chloride; cyclodextrins such as alpha-, beta- or gamma-cyclodextrin; sucrose or glucose esters or derivatives thereof; sulfosuccinates such as dioctyl sodium sulfosuccinate; or combinations thereof.

The composition of the invention may further comprise any other pharmaceutically acceptable excipients commonly present in pharmaceutical formulations. For example, the composition may further comprise an alcohol such as isopropanol, benzyl alcohol, cetearyl alcohol or ethanol; a polysaccharide such as chitosan, chitin, dermatan, hyaluronate, heparin, chondroitin, cyclodextrin or derivatives thereof; or combinations thereof.

Suitable water soluble photosensitizers are readily available from commercial sources, such as Sigma Aldrich, Inc. (Merck KGaA, Darmstadt, Germany), Cayman Chemicals (Ann Arbor, Michigan, USA) and ThermoFisher Scientific (Waltham, MA, USA). For example, riboflavin 5'-phosphate sodium salt, riboflavin 5'-phosphate sodium salt hydrate and riboflavin 5'-phosphate sodium salt dihydrate are available from Sigma Aldrich and Cayman Chemicals. Aqueous compositions, such as solutions, of a photosensitizer, for example a water soluble form of riboflavin, are commercially available or may be prepared using known methods. For example, a water soluble salt, derivative or solvate of riboflavin may be dissolved in an aqueous pharmaceutically acceptable carrier selected from, but not limited to, saline, water, aqueous buffer, an aqueous solution comprising water and a miscible solvent, and combinations thereof. An aqueous composition may additionally include other pharmaceutically acceptable excipients as described above. Photosensitizers formulated as a solution are commercially available, for example ParaCel^{™} (Avedro, Inc, Waltham, MA, USA) is a commercially available aqueous solution comprising 0.25% riboflavin in the form of riboflavin 5'-phosphate sodium salt with hydroxypropylmethylcellulose and benzalkonium chloride, and VibeX Rapid^{™} (Avedro, Inc) is a commercially available solution comprising 0.1% riboflavin 5'-phosphate sodium salt, together with saline and hydroxypropylmethylcellulose. Other commercially available riboflavin solutions include VibeX Xtra^{™} (0.22% riboflavin, saline isotonic); MedioCROSS TE (0.25% riboflavin 5'-phosphate, 1.2% hydroxypropylmethylcellulose, 0.01% benzalkonium chloride); MedioCROSS M (0.1% riboflavin 5'-phosphate, 1.1% hydroxypropylmethylcellulose); Photrexa (0.146% riboflavin 5'-phosphate ophthalmic solution); or Photrexa viscous (0.146% riboflavin 5'-phosphate in 20% dextran ophthalmic solution), all available from Avedro, Inc, Waltham, MA, USA.

In some embodiments the photosensitizer is commercially available as a sterile formulation in a dispenser, for example as a sterile aqueous sodium riboflavin 5'-phosphate solution packaged in a syringe, ampoule, vial or dropper, for example a single use syringe.

The skilled person will understand that the amount of photosensitizer required will depend on the identity of the photosensitizer. In some embodiments the amount of photosensitizer applied to the exposed palpebral conjunctiva is from 1 to 5 mL (and all integers therebetween) of a solution comprising from 0.01 to 0.5% w/v of photosensitizer (and all integers therebetween), suitably 0.1 to 0.25% w/v. In some embodiments the amount of riboflavin applied to an exposed palpebral conjunctiva is 1.8 mL of 0.25% riboflavin (as riboflavin 5'-phosphate) in aqueous carrier; 2 mL of 0.22% riboflavin (as riboflavin 5'-phosphate) in aqueous carrier; 3 mL of 0.146% riboflavin (as riboflavin 5'-phosphate) in aqueous carrier; or 2 mL of 0.1% riboflavin (as riboflavin 5'-phosphate) in aqueous carrier.

The photosensitizer is applied to the exposed palpebral conjunctiva using any suitable means known to the skilled person. In particular embodiments, the photosensitizer is topically applied to the palpebral conjunctiva. In some embodiments, the photosensitizer is applied using an applicator. In some embodiments, the photosensitizer is applied dissolved in an aqueous carrier, preferably as an aqueous solution as discussed herein. In some embodiments, the photosensitizer is instilled directly onto the surface of the exposed palpebral conjunctiva. In some embodiments the photosensitizer is instilled onto the palpebral conjunctiva using, a suitable applicator such as a syringe, pipette or dropper. In alternative embodiments, the photosensitizer solution is first absorbed onto an absorbent material, such as a disposable textile pad, for example a surgical sponge or neurosurgical patty, prior to application to the exposed palpebral conjunctiva. In some embodiments, the photosensitizer is applied to substantially the entire exposed surface of the palpebral conjunctiva.

Optionally, excess photosensitizer may be removed from the palpebral conjunctiva prior to irradiation if desired. For example, excess photosensitizer solution may be removed from the palpebral conjunctiva using absorbent material, such as surgical sponge.

The photosensitizer may be applied to the palpebral conjunctiva simultaneously with or prior to irradiation, especially prior to irradiation. For example, the photosensitizer may be applied from about 30 seconds to about 60 minutes (and all seconds and minutes therebetween) prior to irrradiation, such as from about 1 minute to 60 minutes, 2 minutes to 60 minutes, 2 minutes to 30 minutes, 2 minutes to 20 minutes, 2 minutes to 10 minutes, 2 minutes to 5 minutes and the like, including about 2, 3, 4, 5, 6, 7, 8, 9, 10, 15, 20, 25 or 30 minutes prior to irradiation. In some embodiments, the photosensitizer is applied from about 2, 3, 4 or 5 minutes prior to irradiation. Where required, additional photosensitizer may be applied to the palpebral conjunctiva during irradiation.

In some embodiments, the photosensitizer is allowed to remain in contact with the exposed palpebral conjunctiva for a period of from about 30 seconds to about 60 minutes (and all seconds and minutes therebetween), such as from about 1 minute to 60 minutes, 2 minutes to 60 minutes, 2 minutes to 30 minutes, 2 minutes to 20 minutes, 2 minutes to 10 minutes, 2 minutes to 5 minutes and the like, including about 2, 3, 4, 5, 6, 7, 8, 9, 10, 15, 20, 25 or 30 minutes. In some embodiments, the photosensitizer is allowed to remain in contact with the exposed palpebral conjunctiva for a period of from about 2, 3, 4 or 5 minutes. The exact timing will depend on the amount and concentration of the photosensitizer and can be easily determined by the skilled person based on the concentration of the photosensitizer solution. In some embodiments, the photosensitizer applied to the palpebral conjunctiva is 0.1% riboflavin (as riboflavin 5'-phosphate, sodium salt) in aqueous solution which is allowed to contact the palpebral conjunctiva for approximately 2 to 5 minutes, such as 2, 3, 4 or 5 minutes. In some embodiments, the photosensitizer applied to the palpebral conjunctiva is 0.25% riboflavin (as riboflavin 5'-phosphate, sodium salt) in aqueous solution which is allowed to contact the palpebral conjunctiva for approximately 2 to 5 minutes, such as 2, 3, 4 or 5 minutes. Additional photosensitizer solution may be applied during this time, for example, to ensure that the exposed palpebral conjunctiva is wet and covered with the solution for the desired duration.

After the photosensitizer has contacted the palpebral conjunctiva for the required length of time, the absorbent material, or excess solution, is optionally removed and the exposed palpebral conjunctiva is exposed to radiation of appropriate wavelength for the photosensitizer used. Suitable wavelengths are discussed *supra.* In particular embodiments, the photosensitizer is a sodium salt of riboflavin 5'-phosphate and the radiation wavelength is in the range of from about 300 to about 500 nm (and all integers therebetween) or from about 300 and about 400 nm, especially UV-A radiation of a wavelength of about 320 to about 400 nm. In preferred embodiments, the radiation wavelength is about 365 nm.

The skilled person will understand that the irradiation time necessary to induce sufficient crosslinking will be dependent on several factors including the irradiance intensity delivered by the radiation source (mW/cm²), and the beam width. Preferably, use of an irradiance of about 3 mW/cm² up to about 150 mW/cm² is envisaged. It will also be appreciated that the radiant exposure should not be detrimental to the health of the tissue, particularly the palpebral conjunctiva. A radiant exposure, or fluence, of about 4 to about 27 J/cm² is considered appropriate. In some embodiments, the radiant exposure of the palpebral conjunctiva, or fluence, is from about 5 to about 8 J/cm². In some embodiments, the fluence is about 27 J/cm². The skilled person will be able to determine the duration of the exposure required based on the power of the radiation.

Suitable durations may include, but are not limited to, from about 10 seconds to 30 minutes (and all integer seconds and minutes therebetween), about 30 seconds to 20 minutes, 1 minute to 10 minutes or 3 minutes to 7 minutes. In some embodiments, the duration of exposure is about 1, 2, 3, 4, 5, 6, 7, 8, 9, 10, 11, 12, 13, 14 or 15 minutes. In some embodiments, the duration of exposure is about 3 minutes.

Suitably, the palpebral conjunctiva is exposed to UV-A radiation (320 nm to 400 nm, for example 365 nm) at an irradiance of about 3 to about 6 mW/cm², for example about 3 mW/cm² or about 6 mW/cm².

In some embodiments, the irradiation may be carried out at high irradiance, for example at 30-45 mW/cm² for 1 to 4 minutes, for example 2 to 3 minutes. In some embodiments the radiant exposure, or fluence, is from about 5 to 8 J/cm², for example from about 5.4 to 7.2 J/cm².

The skilled person will appreciate that there will be an upper limit to the amount of irradiation that is considered useful. It is considered that irradiation levels of up to and including 150 mW/cm², for example 150 mW/cm², may be used safely. Irradiation at a level of 150 mW/cm² should be carried out for up to about 4 minutes, or up to about 3 minutes; for example 1 to 4 minutes, 1 to 3 minutes, 2 to 3 minutes, 1 to 2 minutes, or about 1, 2 or 3 minutes.

In some embodiments, the radiation is applied using a beam profile of up to about 15 mm (including about 10, 11, 12, 13, 14 and 15 mm), for example, at a distance of from about 10 mm to about 30 mm (and all integer mm therebetween) from the surface of the palpebral conjunctiva. In some embodiments, the radiation is applied using a beam profile of greater than 12 mm at 10 mm from a surface of the palpebral conjunctiva. A skilled person will appreciate that narrower radiation beams, such as about 11 mm, may be used if the radiation source is repositioned at time intervals to ensure that the entire surface of the palpebral conjunctiva is irradiated.

The delivery of the irradiation may be continuous or pulsed, especially continuous. While the entire surface of the exposed palpebral conjunctiva may be irradiated, in some embodiments, the palpebral conjunctiva is irradiated substantially where the photosensitizer has been applied.

The skilled person will readily appreciate that the wavelength of the photo-activating radiation will depend on the photosensitizer used. In some embodiments, the photo-activating radiation is radiation with a wavelength in the range of from about 300 to about 500 nm (and all integers therebetween) or a wavelength in the range of from about 300 to about 400 nm, particularly UV-A radiation or radiation with a wavelength in the range of from about 320 to about 400 nm. In some embodiments, the radiation has a wavelength in the range of from about 330 to 390, 340 to 380, 350 to 370 or 360 to 370 nm. In particular embodiments, the photo-activating radiation is radiation with a wavelength of about 320, 325, 330, 335, 340, 345, 350, 355, 360, 365, 370, 375, 380, 385, 390, 395 or 400 nm; especially about 355, 360, 365, 370 or 375 nm. In particular embodiments, the photo-activating radiation is radiation with a wavelength of about 365 nm.

In some preferred embodiments, an eye shield or protector, such as a metallic eye protector, is placed under the eyelid and against the anterior aspect of the eye globe prior to irradiation to protect the eye globe from radiation.

In further embodiments, the methods comprise irradiation in the presence of one or more crosslinking agents, preferably a pharmaceutically acceptable crosslinking agent. The skilled person will understand that a crosslinking agent will be preferably substantially non-irritant and non-toxic. A skilled person will be well aware of suitable crosslinking agents. In some embodiments, the crosslinking agent is O₂ gas. For example, in some embodiments irradiation is carried out in the presence of O₂ gas to provide O₂ concentrations greater than those present in normal atmospheric conditions or in the palpebral conjunctiva. In some embodiments, the additional O₂ is provided at the site of crosslinking during irradiation. In some embodiments O₂ gas is delivered to the surface of the palpebral conjunctiva in the proximity of the irradiation site, for example using a delivery device such as an applicator. In some embodiments, the O₂ gas is humidified prior to use. Methods of generating humidified O₂ gas are known to the skilled person, and include passing the gas flow through a humidifier prior to delivery to the required site.

Any radiation source that is able to deliver radiation of the selected wavelength is suitable for use in the methods of the invention. Radiation sources are known in the art, and suitable sources are commercially available. Radiation sources include those suitable for or intended for corneal crosslinking procedures such as, for example, XLink^{™} (Optos, Dunfermline, Scotland); Opto XLink^{™} (Opto Electronica, Sao Carlos, Brazil); CBM Vega XLink Crosslinking System (Carleton Optical, Chesham, UK); LightLink CXL^{™} (LightMed, San Clemente, CA, USA); UV-X^{™} 2000 Crosslinking System (IROC Innocross, Zurich, Switzerland); KXL^{™} CrossLinking System (Avedro Waltham, MA, USA); or an Omnicure UV curing system, such as the OmniCure s1500 UV Curing System (Excelitas Technologies Corp., Waltham, MA, USA). Many of these radiation sources typically have a beam width of approximately 10 to 12 mm. If irradiation of a greater area is necessary, a skilled person will be aware that repositioning of the radiation beam may be required or the radiation source instrument may be modified to produce a greater beam width, such as up to about 15 mm.

In preferred embodiments, the photosensitizer initiates crosslinking of collagen in the tarsal plate tissue in response to photo-activating radiation.

In some embodiments, the method comprises, consists or consists essentially of the steps of:
a) anesthetizing an eyelid of the subject;
b) sterilizing the eyelid;
c) everting the eyelid to expose a palpebral conjunctiva of the eye;
d) applying to at least part of the exposed palpebral conjunctiva a photosensitizer that initiates crosslinking in response to photo-activating radiation;
e) applying directly to the exposed palpebral conjunctiva a radiation beam (e.g. UV-A) to effect crosslinking;
f) rinsing the exposed palpebral conjunctiva to remove residual photosensitizer; and
g) reverting the eyelid to its natural position.

In some embodiments, the method comprises the steps of:
a) administering a local anesthetic (e.g. oxybuprocaine 0.4% eye drops) to the surface of an eye of the subject to be treated;
b) cleaning the periocular area of the eye, including the upper and lower eyelids, using a solution (e.g. chlorhexidine 0.1% or iodine 0.5%);
c) placing a corneo-scleral shield over the ocular surface;
d) everting the eyelid to expose a palpebral conjunctiva of the eye (e.g. using a lid retractor);
e) applying an opaque surgical drape with a central aperture (e.g. about 10 x 20 mm in size) over the everted eyelid;
f) applying to the exposed palpebral conjunctiva drops of a photosensitizer that initiates crosslinking in response to photo-activating radiation, and leaving the photosensitizer in contact with the exposed palpebral conjunctiva for 2-5 mins;
g) applying directly to the exposed palpebral conjunctiva a radiation beam (e.g. UV-A) to effect crosslinking;
h) rinsing the exposed palpebral conjunctiva (e.g. with a saline solution) to remove residual photosensitizer;
i) removing the drape and corneo-scleral shield; and
j) reverting the eyelid to its natural position.

In some embodiments, the method comprises, consists or consists essentially of the steps of:
a) performing an ophthalmic examination of the subject (e.g. slit-lamp examination, eyelid eversion, assessment of the palpebral conjunctiva);
b) administering a local anesthetic (e.g. oxybuprocaine 0.4% eye drops) to the surface of an eye of the subject to be treated;
c) placing the subject in a supine position;
d) cleaning the periocular area of the eye, including the upper and lower eyelids, using a sterile solution (e.g. chlorhexidine 0.1% or iodine 0.5%);
e) placing a corneo-scleral shield over the ocular surface;
f) everting the eyelid to expose a palpebral conjunctiva of the eye (e.g. using a sterile lid retractor);
g) applying an opaque sterile adhesive surgical drape with a central aperture (e.g. about 10 x 20 mm in size) over the everted eyelid;
h) applying to the exposed palpebral conjunctiva drops of a sterile photosensitizer solution (e.g. aqueous solution of riboflavin 5'-phosphate monosodium salt) that initiates crosslinking in response to photo-activating radiation to cover the exposed palpebral conjunctiva and leaving the photosensitizer in contact with the exposed palpebral conjunctiva for 2-5 minutes, optionally including applying further drops as needed during the desired time period to keep the exposed palpebral conjunctiva wet and covered with the solution;
i) applying directly to the exposed palpebral conjunctiva a UV-A radiation beam to effect crosslinking, optionally in the presence of O₂ gas delivered close to the irradiation site, for example for 1 to 60 minutes, 6 to 60 minutes, 1 to 6 minutes, 1 to 4 minutes, or about 3 minutes depending on the irradiance level;
j) rinsing the treated exposed palpebral conjunctiva (e.g. with a saline solution) to remove residual photosensitizer;
k) removing the drape and corneo-scleral shield; and
l) reverting the eyelid to its natural position.

In some embodiments, the method further comprises administration of a lubricating eye drop daily (e.g. up to four times daily) for about one week following the procedure. The dry eye symptoms, complications and efficacy of the crosslinking treatment may be assessed at one or more follow up visits to a clinician. For example, the method may further comprise:
a) scheduling and implementing follow-up visits with a clinician at regular intervals, such as at 1 week, 1 month, 3 months and 6 months to monitor progress and assess efficacy;
b) using a blepharotensiometer on both upper and lower eyelids of the patients to determine the effect of the treatment;
c) using diagnostic tests standard in the art to determine the effect on one or more symptoms of dry eye disease, including: ocular surface disease index (OSDI); tear break-up time (TBUT) including fluorescein TBUT and non-invasive TBUT; Schirmer's test; tear osmolarity test; corneal staining; meibography; and patient-reported outcomes; and
d) comparing the results at each visit to determine the efficacy of the treatment.

In some embodiments, the method is performed as follows. A comprehensive ophthalmic examination of the subject is conducted, including a slit-lamp examination, eyelid eversion, and assessment of the tarsal plate and palpebral conjunctiva, to establish the baseline condition and suitability for the procedure. Informed consent may then be obtained from the subject after explaining the procedure, potential risks, benefits, and alternative treatments. A local anesthetic such as oxybuprocaine (0.4%) eyedrops is administered to the ocular surface to achieve effective anesthesia. The subject is then placed a supine position, for example, on a flat operating table with appropriate head support to maintain stability and proper alignment during the procedure. The treatment periocular area, including the upper and lower eyelids, is then cleaned using a sterile solution of, for example, chlorhexidine 0.1% or iodine 0.5%, to minimize the risk of infection and ensure a sterile field. A large, sterile surgical corneo-scleral shield may then be placed over the ocular surface to protect the cornea, conjunctiva and sclera from potential radiation exposure. The treatment eyelid (upper or lower) is everted using a sterile lid retractor to expose the palpebral conjunctiva, facilitating effective application of treatment. A sterile adhesive surgical drape with a central aperture measuring 10 x 20 mm is applied over the everted eyelid. The drape is preferably opaque to prevent the penetration of UV radiation to non-targeted adjacent tissues and ensure precise treatment localization. A sterile photosensitizer solution (e.g. aqueous solution of riboflavin 5'-phosphate monosodium salt) is applied using a sterile dropper to the treatment area and is left in contact with the exposed palpebral conjunctiva for 2-5 mins (e.g. about 3 minutes or about 5 minutes), whilst keeping the treatment area consistently wet and covered with the solution during this time to facilitate optimal conditions for the crosslinking process. A beam of UV-A radiation is then delivered to the treatment area for a duration depending on the irradiance level (e.g. about 10 seconds to 30 minutes, including about 3 minutes) evenly and consistently across the target area to achieve uniform crosslinking. After irradiation, the treatment area is gently rinsed with balanced saline solution to remove residual photosensitizer. The drape and corneo-scleral shield are then removed and the eyelid is reverted to its natural position. The method may further comprise administration of lubricating eye drops up to about four times daily for 1 week to alleviate discomfort and support the healing process.

If required, the methods may be repeated. For example, the methods may be performed one or more times, such as two times, three times or four times over a time period of, for example, 3 months, 6 months, one year, two years, three years and the like.

Additional materials (e.g. surgical materials) are well known to the skilled person and are readily available from commercial sources. Suitable local anesthetics for anesthetizing the eyelid are well known to the skilled person and include, for example, Alcaine^{™} (proparacaine hydrochloride), Naropin^{™} (ropivicane hydrochloride), Marcaine^{™} (bupivacaine hydrochloride) and Novesin^{™} (benoxinate, oxybuprocaine hydrochloride). Suitable antiseptics include Betadine^{™} (povidone/iodine) and Betasept^{™} (chlorhexidine).

In some embodiments, the dry eye disease is accompanied by mild to moderate eyelid laxity. In some embodiments, the subject does not have severe eyelid laxity or eyelid laxity which requires surgical intervention. In some embodiments, the subject does not have eyelid laxity, or does not require treatment for eyelid laxity.

Also provided is a use of a photosensitizer that initiates crosslinking in response to photo-activating radiation in the manufacture of a medicament for treating dry eye disease in a subject, wherein the photosensitizer is to be applied to at least part of an exposed palpebral conjunctiva of an eye of the subject, and the exposed palpebral conjunctiva is to be irradiated with photo-activating radiation to initiate crosslinking in tarsal plate tissue underlying the palpebral conjunctiva.

Suitable embodiments of the photosensitizer, radiation and exposure of the palpebral conjunctiva are as described *supra.* In some embodiments, the use further comprises exposing a palpebral conjunctiva of an eye of the subject prior to application of the photosensitizer.

In another aspect, there is provided a use of a photosensitizer that initiates crosslinking in response to photo-activating radiation for treating dry eye disease in a subject, wherein the photosensitizer is to be applied to at least part of an exposed palpebral conjunctiva of an eye of the subject, and the exposed palpebral conjunctiva is to be irradiated with photo-activating radiation to initiate crosslinking in tarsal plate tissue underlying the palpebral conjunctiva.

Suitable embodiments of the photosensitizer, radiation and exposure of the palpebral conjunctiva are as described *supra.* In some embodiments, the use further comprises exposing a palpebral conjunctiva of an eye of the subject prior to application of the photosensitizer.

In a further aspect, there is provided a photosensitizer that initiates crosslinking in response to photo-activating radiation for use in treating dry eye disease in a subject, wherein the photosensitizer is applied to at least part of an exposed palpebral conjunctiva of an eye of the subject, and the exposed palpebral conjunctiva is irradiated with photo-activating radiation to initiate crosslinking in tarsal plate tissue underlying the palpebral conjunctiva.

Suitable embodiments of the photosensitizer, radiation and exposure of the palpebral conjunctiva are as described *supra.* In some embodiments, the use further comprises exposing a palpebral conjunctiva of an eye of the subject prior to application of the photosensitizer.

Any one of the methods and uses described herein may involve the application of an effective amount of the photosensitizer and photo-activating radiation, or the use of a system or kit as described elsewhere herein.

### 3. Systems and Kits

The invention further provides systems and kits for treatment or inhibition of the progression of dry eye disease.

Accordingly, in another aspect, there is provided a system for or when used for treating dry eye disease comprising an applicator that applies or delivers a photosensitizer to an exposed palpebral conjunctiva of an eye; and a radiation source for providing photo-activating radiation to the palpebral conjunctiva.

Also provided is a kit for or when used for treating dry eye disease comprising an applicator that applies or delivers a photosensitizer to an exposed palpebral conjunctiva of an eye; and a radiation source for providing photo-activating radiation to the palpebral conjunctiva, optionally with instructions to treat dry eye disease.

Suitable photosensitizers, radiation sources and types of photo-activating radiation are as discussed *supra.* In some embodiments, the radiation source provides a beam width of greater than 12 mm at 10 mm from a surface of the palpebral conjunctiva. In some embodiments, the beam width is from 13 mm to 15 mm at a distance of 10 mm from a surface of the palpebral conjunctiva.

Suitable applicators for delivering or applying a photosensitizer to an exposed palpebral conjunctiva of an eye are discussed above. For example, suitable applicators include syringes, droppers, vials, ampoules, pipettes and absorbent materials such as surgical sponges and neurosurgical patties.

The system or kit may also comprise a photosensitizer or a photosensitizer composition as defined herein.

The system or kit of the present invention may also comprise a delivery device that delivers O₂ gas to the exposed palpebral conjunctiva. In some embodiments the O₂ gas may be delivered using a nozzle, syringe, diffuser or the like. Suitable methods of delivering O₂ to the palpebral conjunctiva are disclosed in, for example, US Patent No. 8,574,277.

In order that the invention may be readily understood and put into practical effect, particular preferred embodiments will now be described by way of the following non-limiting examples.

### EXAMPLES

### EXAMPLE 1 - CROSSLINKING OF SHEEP TARSOCONJUNCTIVAL TISSUE

The ovine upper eyelids were harvested postmortem from five cadaveric sheep and tarsal strips were isolated employing the following procedure. The eyelid was held vertically using a pair of tweezers and a horizontal incision was made along the entire length of the tarsal plate and the overlying palpebral conjunctiva using a No. 10 blade. Vertical incisions were placed at the medial and lateral borders and a horizontal incision was made along the bottom of the tarsal plate. For histological examination, the resulting four tissue specimens were segmented longitudinally into 5 mm samples. Care was taken to avoid crush artefacts of the harvested tarsoconjunctival tissue. For mechanical evaluation, whole tarsal strips were used (6 specimens). Prior to irradiation, the samples were stored in phosphate buffered saline (PBS).

For the crosslinking of the tarsoconjunctival samples, the 365 nm UV radiation was generated by the UV Curing System OmniCure 1500 (Excelitas Technologies Corp., Waltham, MA, USA), while a radiometer Dymax ACCU-CAL 50 (Dymax Corp., Torrington, CT, USA) was used to monitor the irradiance at the exposure site on samples. The required irradiance level was assured by adjusting the distance between the source and the sample's surface.

A 0.1% riboflavin 5'-monophosphate sodium salt (Cayman Chemicals, Ann Arbor, Michigan, USA) in PBS solution was prepared. Each sample was soaked in 0.1% riboflavin 5'-monophosphate sodium salt in PBS for 30 minutes. The samples were then exposed to UV-A radiation (365 nm) for 3 minutes applying a series of different irradiances including 45, 75, 150 or 250 mW/cm², and employing 4 samples for each irradiance, in addition to 4 control samples (not irradiated).

### EXAMPLE 2 - HISTOLOGICAL EVALUATION OF RADIATIVE DAMAGE TO TARSAL CONJUNCTIVA

The wet tissue samples of Example 1 were wiped with paper tissues, and then immersed in aluminium molds containing optimal cutting temperature (OCT) medium (Tissue-Tek, Sakura Finetech USA Inc., Torrance, CA, USA). They were then snap-frozen in a slurry of dry ice/ethanol, and stored at -80°C. During freezing in OCT medium, the samples were oriented to enable sectioning along the vertical axis of the tarsal plate. From each sample, at 200 µm intervals, 3 sections each 5 µm thick were cut using a cryostat (Cryostar NX70, Thermo Scientific, Waltham, MA, USA), then transferred to coated slides (SuperFrost Plus, Menzel-Gläser, Braunschweig, Germany), and stored at -20°C. In an automated Leica ST5010-CV5030 Integrated WorkStation, the sections were stained with hematoxylin-eosin (H&E) and coverslipped. The slides were then scanned with a 40x objective in a Pannoramic Scan II slide scanner (3Dhistech Ltd, Budapest, Hungary), and visualized employing the CaseViewer (v2.4) software.

Damage to the palpebral conjunctiva was noticed histologically only in two samples irradiated at 250 mW/cm². A comparison of palpebral damage between a non-irradiated (control) sample (A) and a sample irradiated at 250 mW/cm² (C) is shown in Figure 2. There was no damage caused by irradiation to meibomian glands. Dense fibrous connective tissue was observed surrounding the normal acini.

### EXAMPLE 3- EVALUATION OF POST-IRRADIATION MECHANICAL ENHANCEMENT OF THE TARSOCONJUNCTIVAL TISSUE

Ovine tarsoconjunctival samples were mechanically evaluated in an Instron Materials Testing System, Model 5943 (Instron, Norwood, MA, USA), equipped with a 50-N load cell. The strips were loaded by pneumatic grips, which were set to a gauge distance of 15 mm and submerged into PBS buffer (pre-heated to 37 ± 2°C) in a BioPuls^{™} unit for the duration of measurements. Tensile preloading of 100 mN was applied with an elongation rate of 0.01 mm/s. After this preconditioning, 15 repeated cyclic tensile loading/unloading tests were performed at 10% displacement (strain) at a rate of 1%/s, and then back to zero. The last cycle was used to calculate Young's modulus (YM) and the stress for each specimen. The specimens were then removed from the Instron tester, soaked in a 0.1% riboflavin 5'-monophosphate sodium salt (Cayman Chemicals, Ann Arbor, Michigan, USA) in PBS solution for 30 min, then exposed to irradiation (at an irradiance of 75 mW/cm² for 3 min) and finally re-evaluated in the same tester. The mean values were calculated from six specimens.

The tensile properties of ovine tarsoconjunctival tissue before and after UV-A irradiation are presented in Figure 3. It is evident that the tarsoconjunctival tissue has enhanced mechanical properties following irradiation.

### EXAMPLE 4 - ASSESSING THE DNA DAMAGE AFTER IRRADIATION OF THE TARSOCONJUNCTIVAL TISSUE

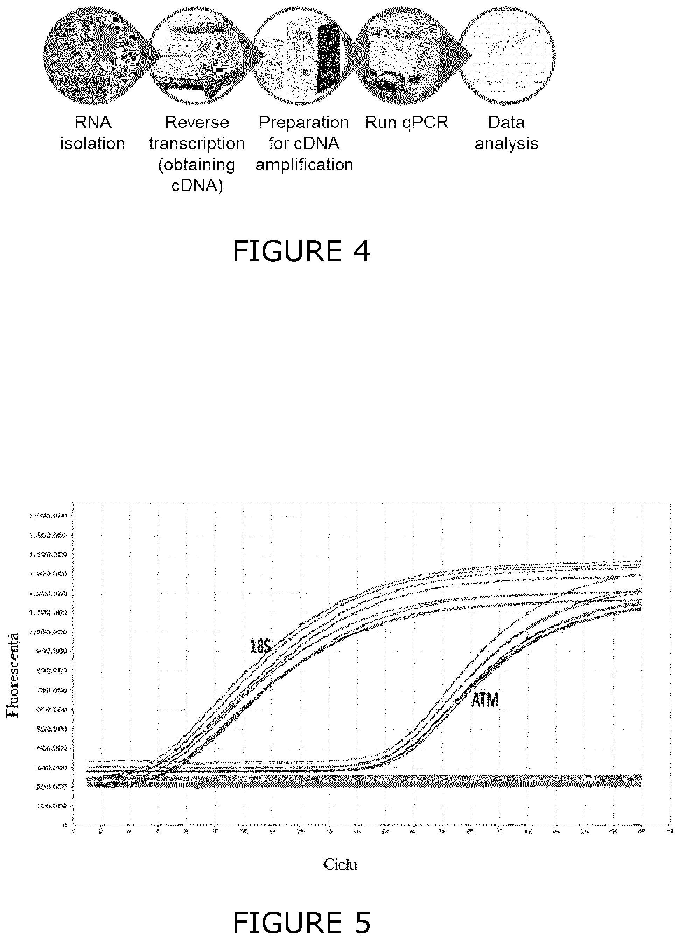

To assess the DNA damage that may be induced by exposure to UV radiation, a gene expression analysis of the ATM (ataxia-telangiectasia, mutated) gene, which has an important role as a potential sensor of DNA damage, was carried out. Real-time PCR (quantitative PCR, qPCR) was performed for the gene expression analysis. The workflow for the analysis is presented in Figure 4.

Ovine tarsoconjunctival eyelid samples that were exposed to UV-A radiation (at an intensity of 45 mW/cm²) and other samples that were not exposed were collected and immediately preserved in an RNA stabilization solution (Invitrogen, ThermoFisher Scientific, Waltham, MA, USA), then stored at -20°C until processing. High-quality purified RNA was isolated from the eyelid samples using mirVana^{™} miRNA Isolation Kit, with phenol (ThermoFisher Scientific). Complementary DNA (cDNA) was obtained through reverse transcription using High Capacity cDNA Reverse Transcription Kit (ThermoFisher Scientific) and Mastercycler^{™} Nexus Thermal Cycler (Eppendorf Austria GmbH, Wien, Austria). For the gene expression analysis, the TaqMan^{®} Gene Expression Master Mix, the ATM TaqMan gene expression assay and reference control genes for sheep (18S) were used. The analysis was performed using the 7500 Fast Dx Real-Time PCR Instrument. For data analysis, the 7500 Software was used in order to quantify rapidly and accurately the relative gene expression (amplification plot presented in Figure 5). Figure 5 shows the expression of 18S and ATM genes in the UV-exposed samples, irradiated with an intensity of 45 mW/cm², and in the control samples, non-irradiated.

No significant differences in the expression of the ATM gene or the reference gene 18S were observed between the UV-irradiated and control samples, which denotes that no DNA damage was induced following irradiation.

The disclosure of every patent, patent application, and publication cited herein is hereby incorporated herein by reference in its entirety.

The citation of any reference herein should not be construed as an admission that such reference is available as "Prior Art" to the instant application.

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention. All such modifications and changes are intended to be included within the scope of the appended claims.

## Claims

1. A photosensitizer that initiates crosslinking in response to photo-activating radiation for use in treating dry eye disease in a subject, wherein the photosensitizer is applied to at least part of an exposed palpebral conjunctiva of an eye of the subject, and the exposed palpebral conjunctiva is irradiated with photo-activating radiation to initiate crosslinking in tarsal plate tissue underlying the palpebral conjunctiva.

2. A method of treating dry eye disease in a subject comprising:
a) exposing a palpebral conjunctiva of an eye;
b) applying to at least part of the exposed palpebral conjunctiva a photosensitizer that initiates crosslinking in response to photo-activating radiation; and
c) irradiating the exposed palpebral conjunctiva with photo-activating radiation to initiate crosslinking in tarsal plate tissue underlying the palpebral conjunctiva.

3. The method according to claim 2, wherein the photosensitizer is in solubilized form.

4. The method according to claim 2 or claim 3, wherein the photosensitizer is selected from the group consisting of riboflavin, acridine orange, Quantacure QTX, protoporphyrin IX and pharmaceutically acceptable salts, derivatives and solvates thereof.

5. The method according to any one of claims 2-4, wherein the photosensitizer absorbs radiation at a wavelength in the range of from about 300 to about 500 nm.

6. The method according to any one of claims 2-5, wherein the photosensitizer is riboflavin or a pharmaceutically acceptable salt, derivative or solvate thereof.

7. The method according to any one of claims 2-6, wherein the photosensitizer is riboflavin 5'-phosphate or a pharmaceutically acceptable salt or solvate thereof.

8. The method according to any one of claims 2-7, wherein the photo-activating radiation is radiation with a wavelength in the range of from about 300 to about 500 nm.

9. The method according to claim 8, wherein the photo-activating radiation is UV-A radiation.

10. The method according to claim 8 or claim 9, wherein the photo-activating radiation is radiation with a wavelength of about 365 nm.

11. The method according to any one of claims 2-10, wherein the palpebral conjunctiva is exposed by everting an upper or lower eyelid of the subject to expose the palpebral conjunctiva.

12. The method according to claim 11, wherein the upper or lower eyelid of the subject is everted using a lid retractor.

13. The method according to any one of claims 1-12, wherein the method further includes delivery of O₂ gas at a site of irradiation.

14. The method according to any one of claims 1-13, wherein the dry eye disease is evaporative dry eye disease.

15. Use of a photosensitizer that initiates crosslinking in response to photo-activating radiation in the manufacture of a medicament for treating dry eye disease in a subject, wherein the photosensitizer is to be applied to at least part of an exposed palpebral conjunctiva of an eye of the subject, and the exposed palpebral conjunctiva is to be irradiated with photo-activating radiation to initiate crosslinking in tarsal plate tissue underlying the palpebral conjunctiva.

16. Use of a photosensitizer that initiates crosslinking in response to photo-activating radiation for treating dry eye disease in a subject, wherein the photosensitizer is to be applied to at least part of an exposed palpebral conjunctiva of an eye of the subject, and the exposed palpebral conjunctiva is to be irradiated with photo-activating radiation to initiate crosslinking in tarsal plate tissue underlying the palpebral conjunctiva.
